# EUROPEAN PATENT APPLICATION

(11) **EP 2 990 068 A1**
(43) Date of publication of application: **02.03.2016**
(21) Application number: 14183040.6
(22) Date of filing: 01.09.2014
(51) Int. Cl.: A61M 25/00, A61B 17/00, A61B 34/00, A61M 25/09

(54) **A device for facilitating the insertion of an endovascular element over a guide-wire**

(71) Applicant: Cabinet Cardiologie Champel SA, 1206 Geneva (CH)
(72) Inventor: Perret, Xavier, 1206 Geneva (CH); Bonvini, Robert Francis, 1224 Chêne-Bougeries (CH)
(74) Representative: Bugnion Genève

(57) **Abstract**

A device (100) for facilitating the insertion of an endovascular element over a guide-wire.

## Description

### FIELD OF THE INVENTION

The invention relates to the field of medical devices. The invention concerns in particular a device for facilitating the insertion of an endovascular element over a guide-wire.

### BACKGROUND OF THE INVENTION

Cardiovascular diseases are the worldwide leading cause of mortality with more than 17 million deaths per year, thus more than 30% of the total mortality.

Ischemic coronary artery diseases (CAD) represent the eighth cause of mortality in the world, and due to the continuous aging of the population, especially in the western countries, CAD related mortality continues to increase.

Treatments of cardiovascular diseases have mainly been developed in the last 30 years. Accordingly, thanks to the development of different types of endovascular percutaneous techniques, the introduction of the interventional cardiovascular medicine has drastically changed the treatments' approaches of all cardiovascular diseases.

The main stem of all endovascular techniques is first to insert into the body, through the skin (e.g., through the groin or the wrist), a small catheter (i.e., long pre-shaped "plastic" tube). Once placed in the vessel, the catheter can be directed, under X-Ray guidance, everywhere in the vascular system in order to reach the target organ (e.g., heart, brain, kidney, etc.) and start the endovascular treatment.

Most of these endovascular techniques are nowadays used to treat atherosclerotic coronary or vascular narrowing (i.e., stenosis or occlusions).

Once, placed just in front of the target vessel, a contrast dye is injected through the catheter in order to correctly visualize the anatomy of the diseased vessel. Always under X-Ray guidance, a small guide-wire is passed through the stenosis in order to reach the distal and healthy part of the vessel.

The operator (i.e., interventional cardiologist, radiologist, vascular specialist, etc.) is now ready to insert through the catheter and over the guide-wire different types of endovascular elements (e.g., balloons, stents [metallic devices], etc.). Most of these elements have the same aim: to dilate the atherosclerotic narrowing and to allow a better blood circulation through the vessel, thus finally improving the organ function.

Thanks to the miniaturization of the material, more and more complex lesions, more and more far away from the guiding catheter inserted through the skin, can nowadays be safely and efficaciously treated. These technical improvements have contributed to a tremendous increase in the use of all endovascular techniques to treat virtually all existing cardiovascular diseases.

The connection of all these endovascular elements over the guide-wire is a mandatory step in order to facilitate the navigation of the element (e.g.,balloon/stent) through the catheter and finally through the vessel's lesion.

According to the size of the used guide-wire (e.g., 0.014"- 0.018"- 0.035" inches) this step may be technically demanding, because:
- All the endovascular elements tend to be as small as possible in order to treat the same lesion through a smaller insertion catheter, thus diminishing the vascular complication rate at the entry puncture site,
- In order to better visualize the screen where the X-Ray images are reproduced, most of the time, operators work in a quite dark/somber environment,
- The visual fine acuity of "senior" operators are relatively reduced (i.e., presbitya), especially if one considers that most of them wear special X-Ray protection glasses and not their usual vision glasses,
- Especially in an emergency setting, with unstable and moving patients, every second is very important in order to reduce the ischemic time of the suffering organ.

It is thus technically difficult to insert an endovascular element (e.g., balloon/stent) over the guide-wire and, to the best of the applicant's knowledge, there is so far no device that is specifically built to perform this operation.

### SUMMARY OF THE INVENTION

The first goal of the invention is to provide a simple medical device in order to facilitate this repetitive manoeuvre (i.e., balloon/stent insertion over the wire performed several times every intervention). In other terms, the first goal of the invention is to provide a device that facilitates the insertion of an endovascular element over a guide-wire.

A second goal of the invention is to provide a device that will improve the manual ability of the operator by performing this endovascular element/guide-wire insertion operation, thus accelerating his manual skill in this particular manoeuvre, thus reducing the time of the intervention, finally improving the patient's and operator's comfort.

A third goal of the invention is to provide a device for facilitating the insertion of an endovascular element over a guide-wire that is easy to use and not expensive, in particular a device which use does not induce any cumbersome manipulation for being ready to perform the operation which is intended to perform. Furthermore, the device must be usable in a relatively dark/somber environment such as an operating room dealing with X-ray images.

A fourth goal of the invention is to provide a device which is adapted to be used independently from the flatness of area on which it is put. In particular, it is intended to provide a device which is stable even when it is put on an uneven area such as a moving patient body.

A fith goal of the invention is to be built as smooth as possible in order to facilitate the glide of the endovascular element over the guide-wire. The invention must be sufficiently rigid, in order to avoid deformation/kink/rupture when used during an endovascular intervention.

These goals are achieved by a device as set out in claim 1.

Advantageously, the shape of device is such that it renders said device able to provide a guiding support that facilitates manipulation of an endovascular element and a guide-wire in such a way that said endovascular element and said guide-wire slide towards each other along a common straight line.

Additional advantageous features are set out in the dependent claims.

### BRIEF DESCRIPTION OF THE DRAWINGS

The present invention will be better understood by the person skilled in the art when provided with the detailed description below and the accompanying drawings in which:
- Figure 1 is a perspective view of the device in accordance with a preferred embodiment of the present invention;
- Figure 2 is a side view of the device in accordance with a preferred embodiment of the present invention;
- Figure 3 is a front view of the device in accordance with a preferred embodiment of the present invention.

### DETAILED DESCRPTION OF PREFERRED EMBODIMENTS

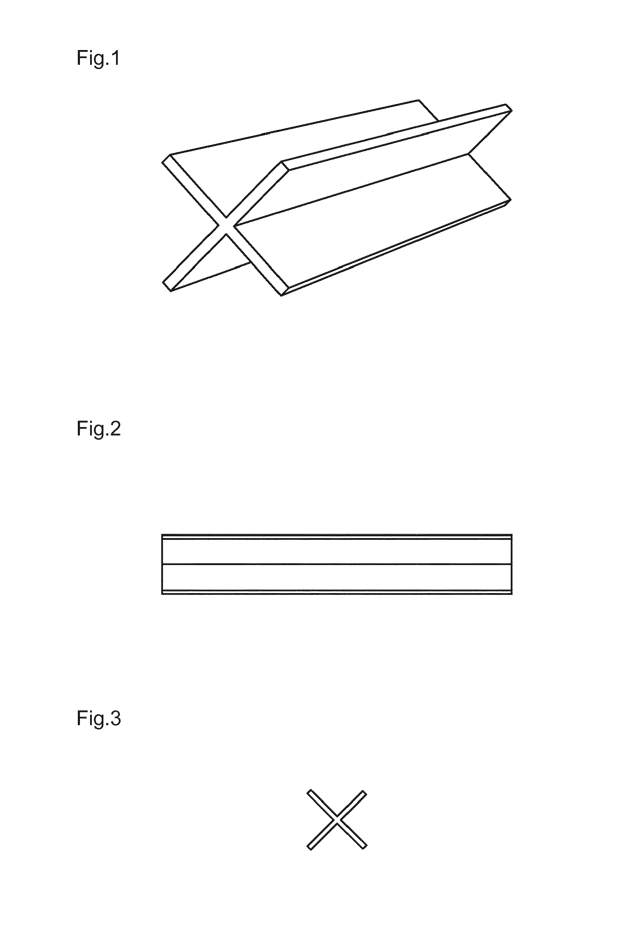

The figures 1-3 show a device for facilitating insertion of a medical endovascular device over a guide-wire according to the preferred embodiment of the invention.

Figure 1 is a perspective view of the device according to the preferred embodiment of the invention.

The device comprises a first wall 101 and a second wall 102. One edge of the first wall 101 is connected to one edge of the second wall 102. The first wall 101 and the second wall 102 are both connected in such a way that an empty space 103 is present between the first wall 101 and the second wall 102. The connection of the first wall 101 and the second wall 102 defines a straight axis 104.

According to the preferred embodiment, the empty space 103 has a substantially prismatic shape and the angle α defined between the first wall 101 and the second wall 102 is equal to 90°. In other embodiments, theangle α is comprised between 30° and 120 ° . Alternatively, the angleα is comprised between 80 and 100.

The device according to the preferred embodiment of the invention further comprises a third wall 105 and a fourth wall 106. The third wall 105 and the fourth wall 106 are both connected by one of their edges to one edge of the first wall 101 and one edge of the second wall 102.

Thus, according to the preferred embodiment of the invention, the device has a profile which has a shape of an "X" or a cross. Thus, an empty space is present between the first wall 101 and the second wall 102, between the second wall 102 and the fourth wall 106, between the third wall 105 and the fourth wall 106 and between the third wall 105 and the first wall 101. Furthermore, each one of the empty spaces identified above has a substantially prismatic shape.

In other alternative embodiments, only the first 101 and the second 102 walls are present. In this case, the rest of the device may have any kind of shape. In other alternative embodiments, the shape of the empty space 103 and/or the empty spaces identified above is curved.

According to the preferred embodiment, the length L of the device is comprised between 2.5 cm and 30 cm and the weight of the device is comprised between 5g and 500g.

The device according to the invention is made in a material that is sterilizable.

As described above, the device according to the invention has a specific shape which is appropriate for drastically facilitating the insertion of an endovascular element over a guide-wire. Indeed, a process of inserting an endovascular element over a guide-wire precisely requires both elements to slide towards each other along a common straight and smooth line. Now, the shape of the device according to the invention is precisely such that the device provides an appropriate support for easily manipulating an endovascular element and a guide wire in such a way that they slide towards each other along a common straight and smooth line.

Indeed, if the distal portion of an endovascular element is inserted from one side of the device while the distal portion of a guide-wire is inserted on the other side of the device, an operator, by pushing both distal ends against the straight axis 104 can make easily both distal portions to slide towards each other along the straight axis 104. When both distal portions meet while the operator maintains a pushing force over the distal portions in the direction of the straight axis 104, the endovascular element is naturally incited to be inserted over the guide-wire.

Moreover, the shape of the device according to the preferred embodiment of the invention makes the device such that it can be easily used in a dark/somber environment such as an operating room dealing with X-Ray images. The operator only needs to drop the device on any surface and the device can immediately be used to perform the operation which is intended to perform.

Finally, the shape of the device, in particular the X-shaped profile of the device, makes the device such that, once dropped on any surface, even an uneven surface such as a moving patient body, it remains sufficiently stable to be safely and efficaciously used.

## Claims

1. A device (100) for facilitating the insertion of an endovascular element over a guide-wire, **characterized in that** it comprises a first wall (101) and a second wall (102), one edge of said first wall (101) being connected to one edge of said second wall (102) so that an empty space (103) is present between said first wall (101) and said second wall (102) and that a straight axis (104) is created by the connection of said first wall (101) and said second wall (102), thereby rendering said device (100) able to provide a guiding support that facilitates manipulation of an endovascular element and a guide-wire in such a way that said endovascular element and said guide-wire slide towards each other along a common straight axis.

2. The device of claim 1, **characterized in that** said empty space (103) has a substantially prismatic shape.

3. The device according to one of the preceding claims, **characterized in that** the angle (α) between said first wall (101) and said second wall (102) is comprised between 30° and 120°.

4. The device according to one of the preceding claims, **characterized in that** the angle (α) between said first wall (101) and said second wall (102) is comprised between 80 and 100 ° .

5. The device according to one of the preceding claims, **characterized in that** it comprises a third wall (105) and a fourth wall (106), said third and fourth walls being connected to said first (101) and second (102) walls.

6. The device according to one of the preceding claims, **characterized in that** it has a X-shaped profile.

7. The device according to one of the preceding claims, **characterized in that** the length (L) of said device is comprise between 2.5 cm and 30 cm.

8. The device according to one of the preceding claims, **characterized in that** it is made in a material that is sterilizable.

9. The device according to one of the preceding claims, **characterized in that** the weight of said device is comprised between 5g and 500g.
